# EUROPEAN PATENT APPLICATION

(11) **EP 4 681 699 A1**
(43) Date of publication of application: **21.01.2026**
(21) Application number: 24189176.1
(22) Date of filing: 17.07.2024
(51) Int. Cl.: A61K 8/25, A61Q 11/00, A61K 8/365, A61K 8/43, A61K 8/90

(54) **COMPOSITION FOR THE DESENSIBILISATION OF TEETH**

(71) Applicant: Ferton Holding S.A., 2800 Delémont (CH)
(72) Inventor: RIZZI, Simone, 1024 Ecublens (CH); TEE, Hisaschi, 64285 Darmstadt (DE); KRATKY, Julia, 64367 Mühltal (DE); PSCHIERER, Sarah, 68647 Biblis (DE)
(74) Representative: Müller Schupfner & Partner Patent- und Rechtsanwaltspartnerschaft mbB (Muc)

(57) **Abstract**

The invention provides a composition for the desensibilisation of teeth comprising
(a) 5 - 66 wt.-% of a colloidal dispersion of silica particles in water,
(b) 32 - 93 wt.-% water,
(c) 0.01 - 2.0 wt.-% surfactant and
(d) 0.04 - 0.50 wt.-% acid,
wherein the composition is deprived of calcium phosphate glass.

## Description

The invention relates to a composition for the desensibilisation of teeth comprising a colloidal dispersion of silica particles in water and to a process for preparing the composition.

Dentin tubules are microscopic channels that extend from the tooth inside through the dentin and end below the enamel. Dental hypersensitivity may occur when dentin tubules are open as a result of a damaged enamel or gingival recession. Such dental hypersensitivity can be treated by a desensibilisation of the teeth, wherein the tubules are occluded or a sealing film is formed which inhibits stimuli resulting in a reduction or elimination of the hypersensitivity of the teeth.

WO 2021/001529 A1 discloses a product for desensibilisation of the teeth which works as a teeth desensitizer. This product contains two main ingredients, a dispersion of silica particles and calcium phosphate glass. The product is a paste that can be spread on the teeth and exposed dentine. The silica dispersion forms a dried gel layer with fine calcium phosphate glass particles that protects the dentine on the surface and occludes the dentine tubules.

The preparation time of this product, however, is about 14 hours. This time is required to reach a product paste consistency that can be spread during application on the teeth. The first step involves mixing of the colloidal silica dispersion with water for 15 minutes (liquid component). To this liquid component CaP glass is added and stirred for 1 h at 700-750 rpm. Furthermore, diluted HCl is added and the suspension is stirred overnight. Finally, chlorhexidine (CHX) digluconate solution (20%) is added and stirred for 5 minutes. Precipitation occurs and after at least 2 h the mixture solidifies (gelation time).

This prototype described in WO 2021/001529 A1 was successful in occluding the dentine tubules and decreasing hypersensitivity in patients, but a long-term storage was not possible as the product deteriorates after about 30 days room temperature. Therefore, a one-component system was not possible due to the short shelf-life. This makes the product not well handleable by end users. The product has to be prepared by the end user, but the gelation time of 2 hours and the preparation time of 14 hours is too long for a useful product to be handled, in particular by end users.

In view of the above, it is an object of the present invention to provide a composition for the desensibilisation of teeth that is a ready to use one-component composition and that can be long-term stored.

This object is achieved by a composition for the desensibilisation of teeth comprising
(a) 5-66 wt.-% of a colloidal dispersion of silica particles in water,
(b) 32 - 93 wt.-% water,
(c) 0.01 - 2.0 wt.-% surfactant and
(d) 0.04 - 0.50 wt.-% acid,
wherein the wt.-% are based on the total weight of the composition and wherein the composition is deprived of calcium phosphate glass.

It is understood that the wt.-% of all components of the composition sum up to 100%.

The object is also achieved by a process of preparing a composition for the desensibilisation of teeth, comprising the steps that the following components are mixed:
(a) 5-66 wt.-% of a colloidal dispersion of silica particles in water,
(b) 32 - 93 wt.-% water,
(c) 0.01 - 2.0 wt.-% surfactant and
(d) 0.04 - 0.50 wt.-% acid,
wherein the wt.-% are based on the total weight of the composition, and wherein the composition is deprived of calcium phosphate glass.

Preferred embodiments of the invention are subject to the dependent claims as well as the following description.

The composition according to the invention is for use in the desensibilisation of teeth. The composition is obtained by mixing components (a) to (d), wherein the composition comprises no calcium phosphate glass, i.e no calcium phosphate glass is mixed into it. The composition according to the invention is a mixture that can directly be applied to teeth. The application can be accomplished for example with a brush or a cotton swab. A film or gel layer is formed that hardens on the teeth, preferably by drying with additional airflow. This procedure seals the dentine tubules and reduces or stops the dental hypersensitivity.

In the course of the present invention, it has been found that the prior art compositions, as described in WO 2021/001529 A1 are not stable due to a drop in pH which is about 0.2 per week at room temperature. This drop in pH has been found being dependent on the calcium phosphate glass concentration in the sample so that the calcium phosphate glass particles were identified to be responsible.

Further investigation revealed that the pH drop was a result of dissolution of the calcium phosphate glass in the aqueous environment over the time which produces acidic substances. Therefore, calcium phosphate glass was excluded from the composition according to the invention and it has been found that additionally the pH needs to be fine-tuned by slightly enhancing the amount of acid. The result is preferably a slightly reduced pH.

Surprisingly, the composition according to the invention is stable and can be long-term stored without significant degradation. Further, it has surprisingly been found that the additional surfactant improves gelation and stability of the composition according to the invention. This makes the composition very useful for the desensibilisation of teeth, for example at the dentist or an end user.

The composition according to the invention is deprived of calcium phosphate glass. That means according to the invention that the composition contains less than 0.1 wt.-% calcium phosphate glass, preferably less than 0.01 wt.-% calcium phosphate glass, based on the total weight of the composition.

"Deprived of" according to the invention can also be named "free of", "not comprising" or "not containing".

In a preferred embodiment of the invention, the composition is essentially consisting of components (a) to (d), preferably consisting of components (a) to (d). That means, a preferred embodiment of the invention is
a composition for the desensibilisation of teeth essentially consisting of, more preferably consisting of
(a) 5-66 wt.-% of a colloidal dispersion of silica particles in water,
(b) 32 - 93 wt.-% water,
(c) 0.01 - 2.0 wt.-% surfactant and
(d) 0.04 - 0.50 wt.-% acid,
wherein the wt.-% are based on the total weight of the composition. The composition is deprived of calcium phosphate glass, in particular deprived of calcium phosphate glass particles.

Calcium phosphate glass is described, for example in in WO 2021/001529 A1. Calcium phosphate glass can be prepared as follows: CaCOs and P₂O₂ are mixed, for example as powders or a powder is prepared in a mortar, the resulting powder is melted in a furnace, for example at around 1000 °C, the molten glass is then poured into water to obtain calcium phosphate glass. The glass can then preferably ground and optionally sieved to have glass particles with a certain maximum particle size, for example preferably < 100µm.

The pH of the composition according to the invention is preferably 2.0 to 8.0, more preferably 5.4 to 7.2, even more preferably 5.5 to 6.5, even more preferably 6.0 - 6.5, most preferably 6.2 - 6.4. These pH values allow a good gelation and a long-term stable composition. Further, the more preferred moderate pH values allow a particularly safe application for end users on the teeth.

The colloidal dispersion of silica particles in water can also be named a silica sol. Such colloidal dispersion of silica particles in water is commercially available, for example under the name KÖSTROSOL^{®}, manufactured by the company Bad Köstritz GmbH. This product KÖSTROSOL^{®} usually comprises 15 - 50 wt.-% amorphous silica and 44.5 - 84.5 wt.-% water and can be further diluted with water.

In a preferred composition according to the invention, the colloidal dispersion of silica particles in water, i.e. component (a), comprises 15 - 50 wt.-% silica and 44.5 - 84.5 wt.-% water, wherein the wt.-% are based on the total weight of the colloidal dispersion of silica particles in water.

Accordingly, a preferred embodiment according to the invention is a composition for the desensibilisation of teeth comprising
(a) 5-66 wt.-% of a colloidal dispersion of silica particles in water, wherein the colloidal dispersion comprises 15 - 50 wt.-% silica and 44.5 - 84.5 wt.-% water, wherein the wt.-% of the silica particles and the wt.-% of the water are based on the total weight of the colloidal dispersion of silica particles in water,
(b) 32 - 93 wt.-% water,
(c) 0.01 - 2.0 wt.-% surfactant and
(d) 0.04 - 0.50 wt.-% acid,
wherein the wt.-% of components (a) to (d) are based on the total weight of the composition and wherein the composition is deprived of calcium phosphate glass.

In the composition of the present invention, the colloidal dispersion of silica particles, i.e. component (a), is diluted with water as component (b). The composition according to the invention comprises preferably 3 - 33 wt.-%, more preferably 5 - 20 wt.-%, silica particles and 65 - 95 wt.-%, preferably 78 - 93 wt.-% water, and 0.01 - 2.0 wt.-% surfactant and 0.04 - 0.50 wt.-% acid.

A colloid or colloidal dispersion according to the invention is understood as particles that are finely dispersed in the dispersion medium. The size of the particles is preferably in the range of one nanometer to one micrometer. i.e. 1 - 1000 nm. The particles size of the silica particles is preferably determined by sieve analysis, as described below, wherein the particle size distribution is based on the mass.

The particles size is preferably determined by sieve analysis, for example with a sieve tower, wherein the particle size distribution is based on the mass (weight). This means, for example, when d₁₀₀ is less than 130 µm that 100 wt.-% (weight percent) of the particles have a size less than 130 µm. The sieve analysis is described, for example, in the German industry norm DIN 66165.

In a preferred embodiment of the invention, the surfactant is selected from the group consisting of pluronic F127, lecithin, cetylpyridinium chloride (CPC), chlorhexidine (CHX) diacetate, chlorhexidine (CHX) digluconate, olaflur, sodium coco sulfate (SCS) or mixtures thereof. The surfactant is selected from the group consisting of pluronic F127, cetylpyridinium chloride (CPC), chlorhexidine diacetate, chlorhexidine digluconate or mixtures thereof. These surfactants have found to give together with an acid a good gelation and a long-term stability of the composition according to the invention.

The amount of the surfactant in the composition of the invention is 0.01 - 2.0 wt.-%, preferably 0.05 - 1.5 wt.-%, more preferably 0.1 - 1.0 wt.-%.

The acid can be an inorganic acid or an organic acid. An organic acid according to the invention is an acid that has a carbon skeleton, for example acetic acid or lactic acid. Preferred organic acids are organic carboxylic acids and organic sulfonic acids, more preferred are organic carboxylic acids.

The inorganic acid is preferably HCl, H₂SO₄ and/or H₃PO₄, more preferably HCl. The organic acid is preferably selected from the group consisting of lactic acid, gluconic acid, tartaric acid, citric acid, alginic acid, glycolic acid, aspartic acid, ascorbic acid, sorbic acid, or mixtures thereof. More preferably, the organic acid is selected from the group consisting of lactic acid and gluconic acid.

The amount of the acid in the composition of the invention is 0.04 - 0.5 wt.-%, preferably 0.08 - 0.4 wt.-%, more preferably 0.11 - 0.38 wt.-%.

In a preferred composition of the invention, component (d) is 0.04 - 0.35 wt.-% inorganic acid, preferably 0.04 - 0.20 wt.-%, more preferably 0.09 - 0.16 wt.-% inorganic acid, or 0.10 - 0.50 wt.-% organic acid, preferably 0.14 - 0.50 wt.-% organic acid.

In a preferred embodiment of the invention, the composition comprises
(a) 14 - 50 wt.-% of a colloidal dispersion of silica particles in water,
(b) 49 - 85 wt.-% water,
(c) 0.01 - 2.0 wt.-% surfactant, preferably 0.1 - 1.0 wt.-% surfactant and
(d) 0.11 - 0.50 wt.-% acid, preferably 0.11 - 0.40 wt.-% acid,
wherein the wt.-% are based on the total weight of the composition and wherein the composition is deprived of calcium phosphate glass, in particular wherein the composition is deprived of calcium phosphate glass particles.

The composition according to the invention can be presented as a kit for use in the desensibilisation of teeth. Accordingly, the invention also relates to a kit for use in the desensibilisation of teeth comprising
A) a first component comprising
   a) 5 - 66 wt.-% of a a colloidal dispersion of silica particles in water,
   b) 32 - 93 wt.-% water, and
B) a second component being a composition, optionally presented in two or more parts, comprising
   c) 0.01 - 2.0 wt.-% surfactant and
   d) 0.04 - 0.50 wt.-% acid,

wherein the composition is deprived of calcium phosphate glass, in particular
wherein the composition is deprived of calcium phosphate glass particles.

The invention also relates to the use of the kit according to the invention for preparing a composition for the desensibilisation of teeth.

The invention also relates to a method (process) for preparing a composition for the desensibilisation of teeth comprising the steps, preferably essentially consisting of the steps, that the following components are mixed:
(a) 5-66 wt.-% of a colloidal dispersion of silica particles in water,
(b) 32 - 93 wt.-% water,
(c) 0.01 - 2.0 wt.-% surfactant and
(d) 0.11 - 0.50 wt.-% acid,
wherein the wt.-% are based on the total weight of the composition, and wherein the composition is deprived of calcium phosphate glass. That means, calcium phosphate glass, in particular calcium phosphate glass particles, are not mixed into the composition.

The following examples provide preferred embodiments according to the invention and further exemplify the invention.

### Examples

The following compositions were prepared:

### List of ingredients

| **Components (concentration of used component in end formulation)** | **One-component system with CaP glass (not according to the invention)** | **One-component system (according to the invention)** | |
|---|---|---|---|
| | **Formulation 1** | **Formulation 2 (CHX with HCl)** | **Formulation 3 (Pluronic with glycolic acid)** |
| Kostrosol | 30.8% | 31.4% | 33.0% |
| Water | 61.1% | 62.7% | 66.0% |
| CHX-Digluconat (20%, solution) | 1% | 0.7% | - |
| HCl(2.14 wt%) | 4.8% | 5.2% | - |
| Lactic acid | - | - | - |
| Glycolic acid | - | - | 0.34% |
| CaP glass | 2.3% | - | - |
| Pluronic F127 | - | - | 1% |
| KCl | - | - | - |
| CPC | - | - | - |
| CHX diacetate | - | - | - |
| Comments | Not stable after 30 days and no microbial stability. | No stability issues (long-term stable and no microbial stability problems) | No stability issues (long-term stable and no microbial stability problems) |

| | | | |
|---|---|---|---|
| Formulations 2 and 3 show a good long-term storability without degradation. | | | |

## Claims

1. Composition for the desensibilisation of teeth comprising
(a) 5 - 66 wt.-% of a colloidal dispersion of silica particles in water,
(b) 32 - 93 wt.-% water,
(c) 0.01 - 2.0 wt.-% surfactant and
(d) 0.11 - 0.50 wt.-% acid,
wherein the wt.-% are based on the total weight of the composition and
wherein the composition is deprived of calcium phosphate glass.

2. Composition according to claim 1, **characterized in that** the pH of the composition is 5.5 to 6.5

3. Composition according to claim 1 or 2, **characterized in that** the acid is an inorganic acid or an organic acid.

4. Composition according to claim 3, **characterized in that** the inorganic acid is HCl.

5. Composition according to claim 3, **characterized in that** the organic acid is selected from the group consisting of lactic acid, gluconic acid, tartaric acid, citric acid, alginic acid, glycolic acid, aspartic acid, ascorbic acid, sorbic acid, or mixtures thereof.

6. Composition according to claim 3, **characterized in that** the organic acid is selected from the group consisting of gluconic acid and lactic acid.

7. Composition according to any of the preceding claims, wherein the surfactant is selected from the group consisting of pluronic F127, lecithin, cetylpyridinium chloride (CPC), chlorhexidine diacetate, chlorhexidine digluconate, olaflur, sodium coco sulfate (SCS) or mixtures thereof.

8. Composition according to any of the preceding claims, **characterized in that** the surfactant is selected from the group consisting of pluronic F127, cetylpyridinium chloride (CPC), chlorhexidine diacetate, chlorhexidine digluconate or mixtures thereof.

9. Composition according to any of the preceding claims, **characterized in that** the composition comprises
(a) 14 - 50 wt.-% of a colloidal dispersion of silica particles in water,
(b) 49 - 85 wt.-% water,
(c) 0.1 - 1.0 wt.-% surfactant and
(d) 0.11 - 0.40 wt.-% acid,
wherein the wt.-% are based on the total weight of the composition and
wherein the composition is deprived of calcium phosphate glass.

10. Composition according to any of the preceding claims, **characterized in that** colloidal dispersion of silica particles in water comprises 15 - 50 wt.-% silica and 44.5 - 84.5 wt.-% water, wherein the wt.-% are based on the total weight of the colloidal dispersion of silica particles in water.

11. Composition according to any of the preceding claims, **characterized in that** component (d) is 0.04 - 0.35 wt.-% inorganic acid or 0.10 - 0.5 wt.-% organic acid.

12. Process of preparing a composition for the desensibilisation of teeth according to any of claims 1 - 11, comprising the steps that the following components are mixed:
(a) 5 - 66 wt.-% of a colloidal dispersion of silica particles in water,
(b) 32 - 93 wt.-% water,
(c) 0.01 - 2.0 wt.-% surfactant and
(d) 0.04 - 0.50 wt.-% acid,
wherein the wt.-% are based on the total weight of the composition.
